(19) Europäisches Patentamt
European Patent Office
Office européen des brevets

(11) EP 0 601 345 B1

(12) EUROPÄISCHE PATENTSCHRIFT

(45) Veröffentlichungstag und Bekanntmachung des Hinweises auf die Patenterteilung: **02.10.1996 Patentblatt 1996/40**

(51) Int. Cl.[6]: **A61K 31/415**, A61K 31/47

(21) Anmeldenummer: **93118167.1**

(22) Anmeldetag: **10.11.1993**

# (54) Imidazol-1-yl-Verbindung enthaltende Arzneimittel

Medicament containing an imidazol-1-yl compound

Médicament contenant un composé imidazol-1-yl

(84) Benannte Vertragsstaaten:
**AT BE CH DE DK ES FR GB GR IE IT LI LU NL PT SE**

(30) Priorität: **14.11.1992 DE 4238553**

(43) Veröffentlichungstag der Anmeldung: **15.06.1994 Patentblatt 1994/24**

(73) Patentinhaber: **Kali-Chemie Pharma GmbH**
**D-30173 Hannover (DE)**

(72) Erfinder:
- **Eeckhout, Christian**
  **D-31812 Bad Pyrmont (DE)**
- **Preuschoff, Ulf**
  **D-29525 Uelzen (DE)**
- **David, Samuel**
  **D-30559 Hannover (DE)**

(74) Vertreter: **Lauer, Dieter, Dr. et al**
**Solvay Pharma Deutschland GmbH**
**Hans-Böckler-Allee 20**
**30173 Hannover (DE)**

(56) Entgegenhaltungen:
**EP-A- 0 201 165** **EP-A- 0 297 651**

- **ALIMENT. PHARMACOL. THER. Bd. 4, Nr. 2 , April 1990 Seiten 139 - 144 S.GORE ET AL. 'Colonic transit in man is slowed by ondansetron (GR38032F), a selective 5-hydroxytryptamine receptor (type 3) antagonist'**
- **SCAND. J. GASTROENTEROL. Bd. 188, Nr. SUPP , 1991 Seiten 124 - 126 C.B.H.W.LAMERS 'Ondansetron: Effects on Gastrointestinal Motility'**
- **J. PHARMACOL. EXP. THER. Bd. 261, Nr. 1 , April 1992 Seiten 297 - 303 K.MIYATA ET AL. 'Role of the Serotonin3 Receptor in Stress-Induced Defecation'**
- **MAYO CLIN. PROC. Bd. 67, Nr. 8 , August 1992 Seiten 732-738 - 804-806 C.J.STEADMAN ET AL. 'Selective 5-Hydroxytryptamine Type 3 Receptor Antagonism With Ondansetron as Treatment for Diarrhea-Predoninant Irritable Bowel Syndrome'**
- **GUT Bd. 31, Nr. 10 , 1990 Seite A1174 A.PRIOR ET AL. 'Reduction of rectal sensitivity and postprandial motility by granisetron, a 5HT3 receptor antagonist, in patients with irritable bowel syndrome (IBS)'**
- **ALIMENT. PHARMACOL. THER. Bd. 6, Nr. 3 , Juni 1992 Seiten 273 - 289 N.J.TALLEY 'Review article: 5-hydroxytryptamine agonists and antagonists in the modulation of gastrointestinal motility and sensation: clinical implications'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.6/3.4

**Beschreibung**

Die vorliegende Erfindung betrifft die Verwendung von Imidazol-1-yl-Verbindungen zur Herstellung von pharmazeutischen Zubereitungen zur Behandlung von funktionellen gastrointestinalen Störungen im Bereich der unteren Darmwege, und zur Herstellung von für diese Behandlung geeigneten Arzneimitteln.

Der Erfindung liegt die Aufgabe zugrunde, neue pharmazeutische Zubereitungen zur Behandlung von funktionellen Krankheitssymptomen im Bereich der unteren Darmwege zu entwickeln.

Erfindungsgemäß werden zur Herstellung von pharmazeutischen Zubereitungen zur Behandlung von mit erhöhter Schmerzempfindlichkeit und/oder anomal beschleunigter Stuhlpassage im Colonbereich verbundenen funktionellen Störungen der unteren Darmwege in größeren Säugetieren und Menschen Imidazol-1-yl-Verbindungen der allgemeinen Formel I,

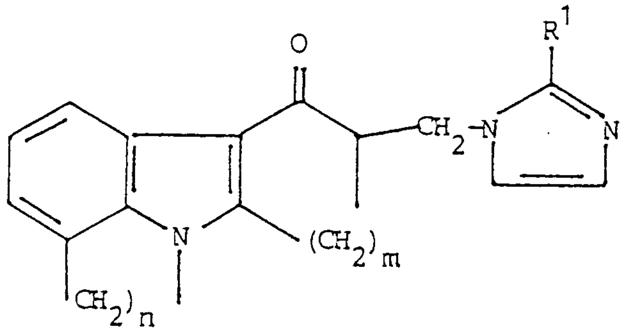

I

worin

$R^1$ niederes Alkyl oder Wasserstoff bedeutet,
m für 2 oder 3 steht und
n für 2 oder 3 steht,

und deren physiologisch verträgliche Säureadditionssalze verwendet.

In den erfindungsgemäß eingesetzten Verbindungen steht m bevorzugt für 2 und n steht bevorzugt für 3. $R^1$ stellt vorzugsweise einen niederen Alkylrest dar, welcher geradkettig oder verzweigt sein kann und 1 bis 4, insbesondere 1 bis 2, Kohlenstoffatome enthalten kann und bevorzugt Methyl ist.

Die Verbindungen der Formel I besitzen an der Verknüpfungsstelle des tetracyclischen Ringgerüstes mit der Imidazol-1-yl-methyl-Seitenkette ein chirales Zentrum, welches jeweils R- oder S-konfiguriert sein kann. Erfindungsgemäß können die einzelnen stereoisomeren Formen der Verbindungen der Formel I oder deren Gemische verwendet werden.

Als geeignet erweisen sich insbesondere 5,6,9,10-Tetrahydro-10-[(2-methyl-1H-imidazol-1-yl)-methyl]-4H-pyrido[3,2,1-jk]carbazol-11(8H)-one, insbesondere das (S)-5,6,9,10-Tetrahydro-10-[(2-methyl-1H-imidazol-1-yl)-methyl]-4H-pyrido[3,2,1-jk]carbazol-11(8H)-on, und deren physiologisch verträgliche Säureadditionssalze.

Als physiologisch verträgliche Säureadditionssalze der Verbindungen der Formel I eignen sich Salze mit anorganischen Säuren, z.B. Halogenwasserstoffsäuren, insbesondere Chlorwasserstoffsäure, oder Schwefelsäure, oder mit organischen Säuren, beispielsweise niederen aliphatischen Mono- oder Dicarbonsäuren wie Essigsäure, Fumarsäure, Weinsäure, Milchsäure, Maleinsäure oder Zitronensäure, oder aromatischen Carbonsäuren wie z. B. Salicylsäure, Dibenzoylweinsäure oder Ditoluoylweinsäure, oder auch Sulfonsäuren wie beispielsweise Niederalkylsulfonsäuren wie Methansulfonsäure oder gegebenenfalls im Benzolring durch Halogen oder niederes Alkyl substituierte Benzolsulfonsäuren wie p-Toluolsulfonsäure.

Die erfindungsgemäß zur Behandlung von funktionellen Störungen der unteren Darmwege eingesetzten Verbindungen fallen unter den Umfang von in der europäischen Patentanmeldung Nr. 0297 651 beschriebenen, ein annelliertes Indolgerüst enthaltenden Verbindungen mit 5-HT-antagonistischen Eigenschaften, und sind aus dieser Patentanmeldung bekannt. Die Verbindungen der Formel I können auf an sich bekannte Weise nach den in der vorgenannten europäischen Patentanmeldung beschriebenen Verfahren oder analog zu diesen Verfahren hergestellt werden.

Es wurde nun überraschenderweise gefunden, daß die erfindungsgemäß verwendete Gruppe der Verbindungen der Formel I nicht nur, wie in der vorstehend zitierten europäischen Patentanmeldung angegeben, für die Behandlung von Erkrankungen im gastroduodenalen Bereich des Magens und des Zwölffingerdarms, also für die Behandlung von Dyspepsien und Emesen, geeignet sind, sondern auch zur Behandlung von Erkrankungen und funktionellen Störungen der unteren Darmwege eingesetzt werden können.

Damit ergeben sich unerwartet Möglichkeiten zur Behandlung von völlig anderen, nicht mit dyspeptischen Zuständen in Zusammenhang stehenden Erkrankungen.

Störungen im Bereich des distalen Dünndarmes sowie erhöhte Schmerzempfindlichkeit bei physiologischen, verdauungsbedingten Dehnungen des Colons können zu Motilitätsstörungen führen, wie sie bei dem sogenannten "irritable bowel syndrom" (= IBS) beobachtet werden. Zu den hierbei auftretenden Symptomen können neben viszeralen Schmerzen und Motilitätsstörungen im unteren Darmbereich auch Störungen des Stuhlgangs, insbesondere anomal beschleunigte Passage des Stuhls im Colon gehören.

Es wurde nun gefunden, daß die Verbindungen der Formel I die viszerale Schmerzempfindlichkeit herabsetzen und dadurch eine durch übersensible Reaktion auf Colon-Dehnung verursachte Inhibierung der Colonmotilität verhindern können. Zusätzlich haben die Verbindungen eine verzögernde Wirkung auf die Stuhlpassage durch das Colon, wodurch sie eine anomal beschleunigte Colonpassage normalisieren können.

Die pharmakologischen Wirkungen der Verbindungen der Formel I im Bereich der unteren Darmwege lassen sich in pharmakologischen Standardtests an Tieren nachweisen.

Beschreibung der pharmakologischen Versuchsmethoden.

I. Untersuchung der Wirkung der Verbindungen auf die Stuhlpassage durch das Colon an der Ratte.

Als Maß für die zur Stuhlausscheidung führende Colonmotilität wird die mittlere Verweilzeit im Colon von in dieses eingeführtem radioaktiv markiertem Material bis zu seiner Ausscheidung im Kot bestimmt.

Für die Versuche wurden jeweils Gruppen von 8 männlichen Wistar-Ratten eingesetzt. Unter Ketamin-Anästhesie wurde den Tieren ein kleines Polyethylenkatheter vom Nacken aus in die Bauchhöhle eingeführt und von dort aus in das Lumen des proximalen Colons 2 cm von der Einmündung des Cökums in das proximale Colon entfernt eingeführt.

Durch das Katheter wurde den Tieren je 0,1 ml einer mit $^{51}$Cr radioaktiv markierten Natriumchromatlösung (= 10 $\mu$ Cr/ml) injiziert. Dann wurden von dem abgehenden Kot in 1-Stunden-Abständen Proben gesammelt solange, bis keine Radioaktivität mehr im Kot nachweisbar war. Die in den Kotproben enthaltene Radioaktivität wurde mit Hilfe eines Gammazählers gemessen. Aus diesen Messungen wurde die mittlere Verweilzeit des radioaktiv markierten Materials im Colon berechnet. Diese mittlere Verweilzeit kann als Maß für die Geschwindigkeit der Colonpassage dienen.

Bei einer Kontrolltiergruppe betrug die mittlere Verweilzeit des $^{51}$Cr-markierten Materials im Colon 7,1 Stunden. Subkutane Applikation von (S)-5,6,9,10-Tetrahydro-10-[(2-methyl-1H-imidazol-1-yl)-methyl]-4H-pyrido[3,2,1-jk]carbazol-11(8H)-on-hydrochlorid in einer Dosis von 1 mg/kg 15 Minuten vor Applikation des radioaktiv markierten Natriumchromates bewirkte eine Verlangsamung der Ausscheidung aus dem Colon und führte zu einer Verlängerung der mittleren Verweilzeit des radioaktiv markierten Materials im Colon auf 9,8 Stunden. Dies zeigt, daß die Testsubstanz die zur Kotausscheidung führende Colonaktivität vermindert.

II. Untersuchung des Einflusses der Verbindungen auf die viszerale Empfindlichkeit an der Ratte.

Männliche Wistar-Ratten mit einem Körpergewicht von 250 bis 350 g wurden durch einen chirurgischen Eingriff für eine Elektromyographie vorbereitet, indem ihnen Elektroden aus Nickelchromdraht in die Wand des proximalen Colons eingesetzt wurden. Die elektromyographischen Aufzeichnungen der Colonbewegungen wurden 5 Tage nach dem chirurgischen Eingriff begonnen. Für die Versuchsdurchführung wurde den Tieren ein dehnbarer Ballon rektal soweit in das Colon eingeführt, daß das Ballonende sich 1 cm vom Rektum entfernt befand, und der Ballon wurde in dieser Position am Schwanz befestigt. Während des Versuches wurden die Tiere in einem tunnelförmigen Käfig gehalten, in welchem sie sich bewegen aber nicht umdrehen oder rauslaufen konnten. Durch Aufblasen des Ballons auf einen Durchmesser von 11 mm wurde die Colonmotilität verringert. Hierbei verringert sich die Anzahl der Kontraktionsserien pro 5 Minuten bei einer Gruppe von Kontrolltieren auf 43 % des ursprünglichen Wertes. Nach ip-Applikation von (S)-5,6,9,10-Tetrahydro-10-[(2-methyl-1H-imidazol-1-yl)-methyl]-4H-pyrido[3,2,1-jk]carbazol-11(8H)-on-hydrochlorid in einer Dosis von 1 mg/kg vor Versuchsbeginn war die Empfindlichkeit gegenüber Dehnung verringert und die Anzahl der Kontraktionsserien pro 5 Minuten wurde nur auf 85 % des ursprünglichen Wertes verringert.

Die vorstehenden pharmakologischen Versuchsergebnisse zeigen, daß die Verbindungen der Formel I die durch Stimulation der afferenten Nerven verursachten Störungen der Colonmotilität verhindern können und deshalb für die Behandlung von IBS geeignet sind. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größeren Säugetieren jedoch Arzneiformen mit einem Wirkstoffgehalt von 0,1 bis 80, insbesondere 1 bis 10 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese Präparate können pharmazeutisch übliche anorganische und/oder organische Trägerstoffe, wie z.B. Talkum, Milchzucker oder Stärke neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalen. Es können zusätzlich weitere Hilfsstoffe zugegeben werden wie z. B. Konservierungsmittel, Geschmackskorregenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die folgenden Beispiele sollen die Herstellung von Verbindungen der Formel I enthaltenden pharmazeutischen Zubereitungen näher erläutern.

<u>Beispiel 1:</u> Tabletten

Zusammensetzung:

| | |
|---|---|
| (S)-5,6,9,10-Tetrahydro-10-[(2-methyl-1H-imidazol-1-yl)-methyl]-4H-pyrido[3,2,1-jk]carbazol-11(8H)-on-hydrochlorid | 5 Teile |
| Maisstärke | 30 Teile |
| Lactose | 70 Teile |
| Kollidon $25^R$ | 5 Teile |
| Magnesiumstearat | 2 Teile |
| Talkum | 3 Teile |
| Gesamt | 115 Teile |

Herstellungsvorschrift:

Der Wirkstoff wird mit Maisstärke und feinpulvriger Lactose in einem Mischer vermischt. Die entstandene Mischung wird mit einer 20%-igen Lösung von Polyvinylpyrrolidon (Kollidon $25^R$ der Fa. BASF) in entmineralisiertem Wasser durchfeuchtet. Falls erforderlich wird weiteres entmineralisiertes Wasser hinzugefügt. Das feuchte Granulat wird durch ein 2 mm-Sieb passiert, bei 40 °C auf Horden getrocknet und anschließend durch ein 1 mm-Sieb (Frewitt-Maschine) passiert. Nach dem Vermischen des Granulates mit Magnesiumstearat und Talkum werden daraus Tabletten mit einem Gewicht von 115 mg gepreßt, so daß jede Tablette 5 mg Wirkstoff enthält.

Beispiel 2: Kapseln

Zusammensetzung:

| | |
|---|---|
| (S)-5,6,9,10-Tetrahydro-10-[(2-methyl-1H-imidazol-1-yl)-methyl]-4H-pyrido[3,2,1-jk]carbazol-11(8H)-on-hydrochlorid | 5 Teile |
| Maisstärke | 20 Teile |
| Lactose | 60 Teile |
| Kollidon $25^R$ | 3 Teile |
| Magnesiumstearat | 1,5 Teile |
| Aerosil $200^R$ | 0,5 Teile |
| Gesamt | 90 Teile |

Herstellungsvorschrift:

Der Wirkstoff wird mit Maisstärke und feinpulvriger Lactose in einem Mischer vermischt. Die entstandene Mischung wird mit einer 20%-igen Lösung von Polyvinylpyrrolidon (Kollidon $25^R$ der Fa. BASF) in entmineralisiertem Wasser durchfeuchtet. Falls erforderlich wird entmineralisiertes Wasser hinzugefügt. Das feuchte Granulat wird durch ein 1,6 mm-Sieb (Frewitt-Maschine) passiert, bei 40 °C auf Horden getrocknet und anschließend durch ein 1 mm-Sieb (Frewitt) passiert. Nach dem Vermischen des Granulates mit Magnesiumstearat und hochdisperser Kieselsäure (Aerosil $200^R$, Fa. Degussa) füllt man jeweils davon 90 mg mittels automatischer Kapselmaschine in Hartgelatinekapseln der Größe 4 ab, so daß jede Kapsel 5 mg Wirkstoff enthält.

## Patentansprüche

1. Verwendung von Imidazol-1-yl-Verbindungen der allgemeinen Formel I

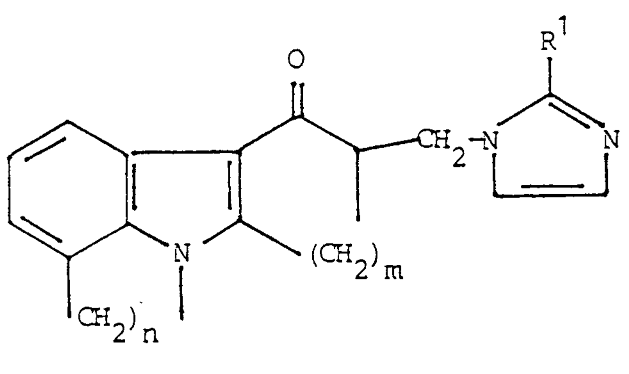

I

worin

$R^1$ niederes Alkyl oder Wasserstoff bedeutet,
m für 2 oder 3 steht und
n für 2 oder 3 steht,

und deren physiologisch verträglichen Säureadditionssalzen zur Herstellung von pharmazeutischen Zubereitungen zur Behandlung von mit erhöhter Schmerzempfindlichkeit und/oder anomal beschleunigter Stuhlpassage im Colonbereich verbundenen funktionellen Störungen der unteren Darmwege in größeren Säugetieren und Menschen.

2. Verwendung nach Anspruch 1, dadurch gekennzeichnet, daß Verbindungen der Formel I eingesetzt werden, worin m für 2 und n für 3 stehen.

3. Verwendung nach Anspruch 2, dadurch gekennzeichnet, daß 5,6,9,10-Tetrahydro-10-[(2-methyl-1H-imidazol-1-yl)-methyl]-4H-pyrido[3,2,1-jk]carbazol-11(8H)-on und dessen physiologisch verträgliche Säureadditionssalze eingesetzt werden.

4. Verwendung nach Anspruch 3, dadurch gekennzeichnet, daß (S)-5,6,9,10-Tetrahydro-10-[(2-methyl-1H-imidazol-1-yl)-methyl]-4H-pyrido[3,2,1-jk]carbazol-11(8H)-on und dessen physiologisch verträgliche Säureadditionssalze eingesetzt werden.

## Claims

1. Use of imidazol-1-yl compounds of the general formula I

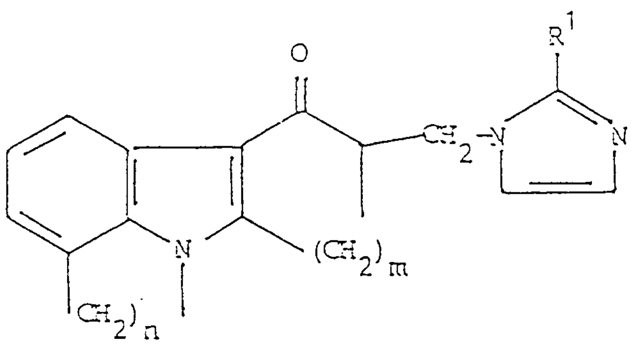

I

wherein

$R^1$ denotes lower alkyl or hydrogen,
m represents 2 or 3 and
n represents 2 or 3,

and physiologically tolerated acid addition salts thereof for the preparation of pharmaceutical formulations for the treatment of functional disturbances of the lower intestinal tract associated with increased pain sensitivity and/or abnormally accelerated stool passage in the colon region in larger mammals and humans.

2. Use according to Claim 1, characterized in that compounds of the formula I wherein m represents 2 and n represents 3 are used.

3. Use according to Claim 2, characterized in that 5,6,9,10-tetrahydro-10-[(2-methyl-1H-imidazol-1-yl)-methyl]-4H-pyrido[3,2,1-jk]carbazol-11(8H)-one and physiologically tolerated acid addition salts thereof are used.

4. Use according to Claim 3, characterized in that (S)-5,6,9,10-tetrahydro-10-[(2-methyl-1H-imidazol-1-yl)-methyl]-4H-pyrido[3,2,1-jk]carbazol-11(8H)-one and physiologically tolerated acid addition salts thereof are used.

## Revendications

**1.** Utilisation de composés imidazol-1-ylique de formule générale I

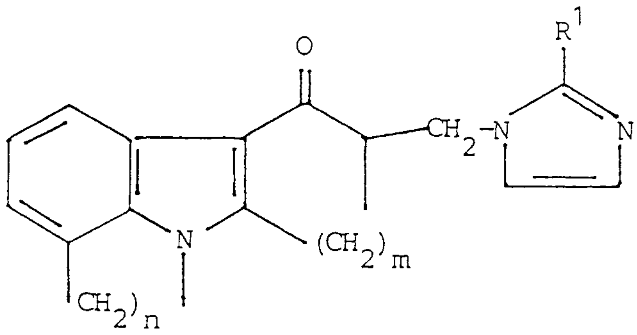

I

dans laquelle

$R^1$ représente un groupe alkyle inférieur ou un atome d'hydrogène ;
m est un nombre valant 2 ou 3, et
n est un nombre valant 2 ou 3,

et de leurs sels d'addition d'acides physiologiquement tolérables, pour produire des préparations pharmaceutiques pour le traitement de troubles fonctionnels des voies intestinales inférieures, ces troubles étant liés à une sensibilité accrue à la douleur et/ou à une accélération anormale du transit des matières dans le colon, chez des mammifères supérieurs et des êtres humains.

**2.** Utilisation selon la revendication 1, caractérisée en ce qu'on utilise des composés de formule I, dans laquelle m vaut 2 et n vaut 3.

**3.** Utilisation selon la revendication 2, caractérisée en ce qu'on utilise la 5,6,9,10-tétrahydro-10-[(2-méthyl-1H-imidazol-1-yl)-méthyl]-4H-pyrido [3,2,1-jk]-carbazol-11(8H)-one et ses sels d'addition d'acides physiologiquement tolérables.

**4.** Utilisation selon la revendication 3, caractérisée en ce qu'on utilise la (S)-5,6,9,10-tétrahydro-10-[(2-méthyl-1H-imidazol-1-yl)-méthyl]-4H-pyrido [3,2,1-jk]-carbazol-11(8H)-one et ses sels d'addition d'acides physiologiquement tolérables.